# EUROPÄISCHE PATENTSCHRIFT

(11) **EP 2 926 746 B1**
(45) Veröffentlichungstag und Bekanntmachung des Hinweises auf die Patenterteilung: **20.12.2017**
(21) Anmeldenummer: 15160714.0
(22) Anmeldetag: 25.03.2015
(51) Int. Cl.: A61B 17/16

(54) **CHIRURGISCHES INSTRUMENT**
SURGICAL INSTRUMENT
INSTRUMENT CHIRURGICAL

(30) Priorität: 01.04.2014 DE 102014104550
(43) Veröffentlichungstag der Anmeldung: 07.10.2015
(73) Patentinhaber: MEDICON EG CHIRURGIEMECHANIKER-GENOSSENSCHAFT, 78532 Tuttlingen (DE)
(72) Erfinder: Burger, Andreas, 78532 Tuttlingen (DE); Widmaier, Gerd, 78532 Tuttlingen (DE); Alber, Matthias, 78587 Irndorf (DE); Wenzler, Martin, 78582 Balgheim (DE)
(74) Vertreter: Mammel und Maser

(56) Entgegenhaltungen:
- US-A- 3 269 630
- US-A- 5 782 749

## Beschreibung

### Chirurgisches Instrument

Die vorliegende Erfindung betrifft ein chirurgisches Instrument mit einem Schaft, der proximal in einen feststehenden Griff mündet und einem Schieber, der auf dem Schaft in Längsrichtung verschiebbar ist, wobei der Schieber mit einem schwenkbaren Handgriff bewegungsgekoppelt ist und wobei der schwenkbare Handgriff mit dem feststehenden Handgriff gelenkig verbunden ist.

Insbesondere betrifft die Erfindung ein Schiebeschaftinstrument, wie beispielsweise eine Knochenstanze, einen Rongeur oder ein Conchotom.

Solche Schiebeschaftinstrumente, die in der Chirurgie seit langem bekannt sind, dienen in der Regel dazu, Gewebe, Knochen oder Knorpel zu durchtrennen und/oder zu entfernen. In der Wirbelsäulenchirurgie werden u. a. Knochenstanzen eingesetzt, die zum exakten Abtragen von Knochen dienen und zum Beispiel bei der Eröffnung des Spinalkanals zum Einsatz kommen.

Ein Schiebeschaftinstrument, das zu Reinigungszwecken einfach zerlegbar ist und bei dem die einzelnen Bestandteile beieinander bleiben, ist beispielsweise aus der DE 10 2009 056 099 B4 bekannt. Dieses Instrument weist am proximalen Ende des Schaftes eine proximale Schaftausnehmung auf, in der ein starr mit dem Schieber verbundener Bolzen verschiebbar geführt ist.

Bei der bislang bekannten Knochenstanze kann es allerdings während der Operation beim Schließen der Maulöffnungen der Knochenstanze dazu kommen, dass benachbartes Gewebe versehentlich herausgestanzt wird. Dies ist insbesondere der Fall, wenn der abzutragende Knochen dünner als die Maulöffnung der Stanze ist. Ist der abzutragende Knochen dicker als die Maulöffnung der Stanze, muss das Instrument zweimal angesetzt werden, was zu einem Zeitverlust während der Operation führt.

Bei solchen Schiebeschaftinstrumenten gibt es im Allgemeinen genau zwei Einstellpositionen, eine Ausgangsposition, in der der schwenkbare Handgriff nicht in Richtung des feststehenden Handgriffs gedrückt ist, das Maul geöffnet und der Hub groß ist und eine Betätigungsposition, in der der schwenkbare Handgriff in Richtung des feststehenden Handgriffs gedrückt, das Maul geschlossen und der Hub minimal ist.

Die RU 2 362 498 D2 lehrt ein chirurgisches Instrument zum automatischen Zuführen und Klammern, bei dem eine Platte mit einem Stab und einem bewegbaren Klauengehäuse durch einen sequentiellen Antriebszyklus zusammenwirkt. Das Klammerinstrument weist einen Zuführmechanismus aus einem bewegbaren Gehäuse auf, das einen Arbeitsstab mit Klauen bedeckt. Mittels eines Nockenprofils können vier verschiedene Positionen eingestellt werden, eine erste Position, in der der bewegbare Körper vorgespannt ist und der Behälter für die Klammern geöffnet ist, eine zweite Position mit geschlossenem Behälter, wobei diese geschlossene Position einer ersten Klammerngröße entspricht, eine dritte Position, die einer anderen Klammerngröße entspricht und einer vierten Position, in der der Schaft von dem Stab entkoppelt ist und der Applikator in seine Komponenten zerlegt werden kann.

Aus der US 3,269,630 ist ein Klammerinstrument bekannt, bei dem die Hubbewegung des bewegbaren Handgriffs gegenüber dem feststehenden Handgriff über einen komplizierten Mechanismus mit einem länglichen Schlitz in dem Schieber, in dem die Drehachse zwischen feststehendem und bewegbaren Handgriff verschiebbar ist und einem bogenförmigen Schlitz in dem bewegbaren Handgriff, in dem der Pin des Kalibrierungsrads positioniert werden kann, einstellbar ist.

Die Aufgabe der Erfindung besteht darin, eine vereinfachte Führung des Schiebers in dem Schaft für Schiebeschaftinstrumente wie Knochenstanzen, bei denen die Weite der Maulöffnung, d.h. der Hub, einstellbar ist, anzugeben.

Diese Aufgabe wird erfindungsgemäß dadurch gelöst, dass am proximalen Ende des Schafts eine proximale Schaftausnehmung, insbesondere ein Langloch, vorgesehen ist, in der ein starr mit dem Schieber verbundener Bolzen verschiebbar geführt ist. Die Einstellvorrichtung, die zwischen dem feststehenden und dem bewegbaren Handgriff wirkt, ist ein Riegel, der die Schiebebewegung des Schiebers oder des mit dem Schieber verbundenen Bolzens, je nachdem, wie weit der Schieber in Querrichtung in den Bewegungsraum des Schiebers hineingeschoben ist, mehr oder weniger beschränkt.

Nachdem die Einstellung des Hubs der Öffnung der Maulweite oder, wenn Werkzeugelemente an den Enden des Schafts und des Schiebers befestigt sind, der Öffnung der Werkzeugelemente entspricht, kann über die Einstellvorrichtung genau eine bestimmte Maulweite oder Öffnungen der Werkzeugelemente eingestellt und die Vorrichtung dann mit dieser Einstellung betrieben werden, bis eine andere Maulweite eingestellt wird.

Die Einstellvorrichtung ist eine Vorrichtung, mit der die Hubbewegung des schwenkbaren Handgriffs zu dem feststehenden Handgriff, d.h. das Ausmaß der Schwenkbarkeit des schwenkbaren Handgriffs zu dem feststehenden Griff bzw. der maximale Öffnungswinkel der Griffe, einstellbar ist. Aus der Betätigungsposition, d.h. wenn der schwenkbare Griff betätigt, d.h. in Richtung des feststehenden Handgriffs gedrückt ist, kommt somit der schwenkbare Griff beim Lösen des schwenkbaren Griffs infolge der Wirkung bzw. der jeweiligen Position der Einstellvorrichtung nicht mehr in seine ursprüngliche Position mit maximalem Hub zurück, sondern wird in einer "neuen" Ausgangsposition mit einer anderen Griffweite als der in ursprünglicher Position, d.h. zum Beispiel mit geringerem Hub des Schiebers (und damit geringer Maulöffnung) und damit mit geringerer Griffweite durch die Einstellvorrichtung festgesetzt. In Abhängigkeit von der Position der Einstellvorrichtung kann somit wahlweise der Hub des Schiebers bzw. die Griffweite zurück in die ursprüngliche Ausgangsposition mit maximalem Hub oder in eine "neue" Ausgangsposition mit geringem Hub eingestellt werden. Durch die Einstellvorrichtung können somit aus der Betätigungsposition wenigstens zwei verschiedene Griffweiten eingestellt werden. Vorzugsweise ermöglicht die Einstellvorrichtung die Einstellung von wenigstens drei und insbesondere vier verschiedenen Griffweiten in der Ausgangsposition.

Die Einstellvorrichtung ist vorzugsweise ein Riegel, der die Schiebebewegung des Schiebers oder des mit dem Schieber verbundenen Bolzens je nachdem, wie weit der Schieber in Querrichtung in den Bewegungsraum des Schiebers hineingeschoben ist, mehr oder weniger beschränkt. Vorzugsweise erfoigt die Beschränkung der Bewegung des Schiebers dadurch, dass der Riegel stufenförmig angeordnete Anschlagflächen aufweist, die in Abhängigkeit von dem Hineinschieben des Riegels in den Bewegungsraum des Schiebers in Querrichtung den Hubweg des Schiebers um die Höhe der jeweiligen Stufe verringern.

Die Erfindung ermöglicht nicht nur eine Einstellung der Maulöffnung bzw. des Hubs, beispielsweise bei Knochenstanzen in Abhängigkeit von der Anatomie, sondern - infolge der Bewegungskopplung von schwenkbarem Griff und Schieber - auch die Einstellbarkeit der Griffweite, beispielsweise für Chirurgen mit kleineren Händen, wie es beispielsweise im asiatischen Raum oder für Frauen erforderlich sein kann.

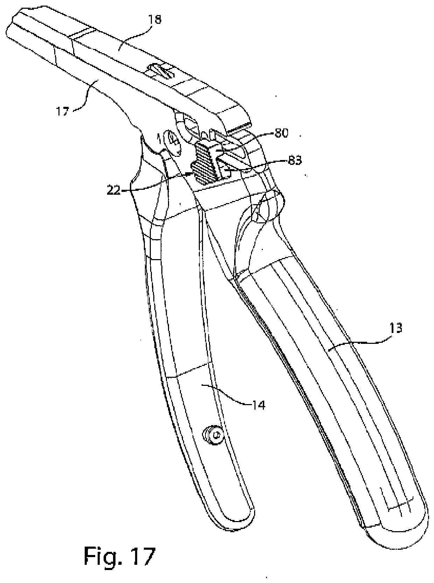

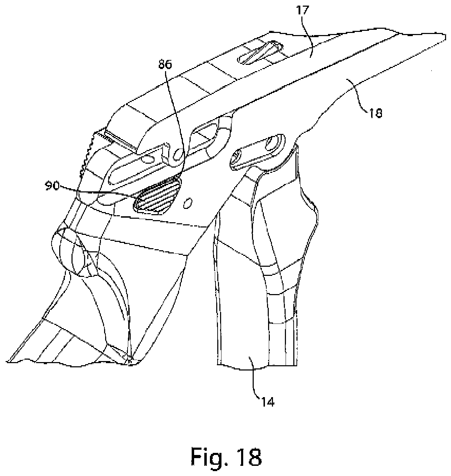

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine Seitenansicht auf die linke Seite eines ersten nicht erfindungsgemäßen chirurgischen Instruments mit einem Drehhebel,
Figur 2 die Seitenansicht auf die rechte Seite des nicht erfindungsgemäßen chirurgischen Instruments aus Fig. 1,
Figur 3 die Ansicht aus Fig. 1, jedoch mit transparentem Schaft 17,
Figur 4 die Ansicht aus Fig. 2, jedoch mit transparentem Schaft 17,
Figur 5 einen Ausschnitt des auf dem Gelenkzapfen 15 gelagerten schwenkbaren Griffs 14, der an der Nocke 42 anschlägt (nicht erfindungsgemäß),
Figur 6 eine perspektivische Ansicht auf das Instrument auf die linke Seite gemäß der Figuren 1 und 3 mit transparentem Schaft 17 (nicht erfindungsgemäß),
Figur 7 eine perspektivische Ansicht in der Ansicht von Fig. 1 nur auf den Schaft 17 und den Schieber 18 (linke Seite) von schräg unten (nicht erfindungsgemäß),
Figur 8 den schwenkbaren Griff 14 in der Ansicht aus Fig. 2 (nicht erfindungsgemäß),
Figur 9 den Gelenkzapfen (Schwenkachse) 15 mit Joch 52 und Entriegelungsbolzen 54 des Instruments aus Figur 1 (nicht erfindungsgemäß),
Figur 10 die Teile aus Figur 9 mit der Einstellvorrichtung, und Entriegelungs-/Zerlegeknopf 39, (nicht erfindungsgemäß)
Figur 10a einen Horizontalschnitt der Teile aus Figur 10 (nicht erfindungsgemäß),
Figur 11 Seitenansicht auf die linke Seite des Schaftes 17 des Instruments aus Figur 1 von schräg unten (nicht erfindungsgemäß),
Figur 12 eine perspektivische Ansicht auf die linke Seite des Schiebers 18 des Instruments aus Figur 1 (nicht erfindungsgemäß),

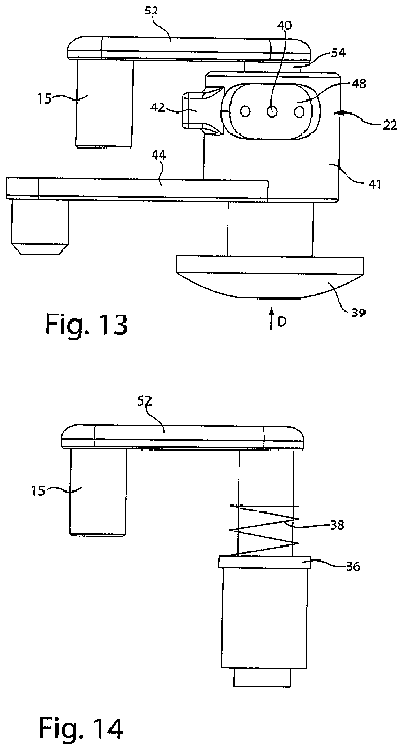

Figur 13 die Draufsicht auf die auf dem Entriegelungsbolzen 54 gelagerte Einstellvorrichtung mit Joch 52 und Schwenkachse 15 des Instruments aus Figur 1,
Figur 14 die Ansicht der Figur 13, aber ohne Einstellvorrichtung,
Figur 15 die Entriegelungsstellung des Instruments aus Figur 1 mit Drehhebel und - zur Erläuterung - ebenfalls dargestellt Verschieberiegel 80 aus Figur 16 (erfindungsgemäß)
   a) in Seitenansicht,
   b) in Richtung des Pfeils A in Figur 15a (von oben),
   c) im Schnitt B-B durch Figur 15a im Bereich des Schieberiegels 80, d.h. den Entriegelungsmechanismus der Ausführungsform gemäß Figur 16-20,
Figur 16 eine erfindungsgemäße Ausführungsform mit einem Verschieberiegel als Einstellvorrichtung (Seitenansicht von links auf den proximalen Bereich des Instruments),
Figur 17 Ansicht auf die linke Seite des Instruments von Figur 16 von hinten in perspektivischer Ansicht,
Figur 18 Ansicht auf die rechte Seite des Instruments aus Figur 16 von hinten in perspektivischer Ansicht,
Figur 19 den Verschieberiegel aus Figur 16 bis 18 und
Figur 20 den Verschieberiegel aus Figur 19 mit Federelement.

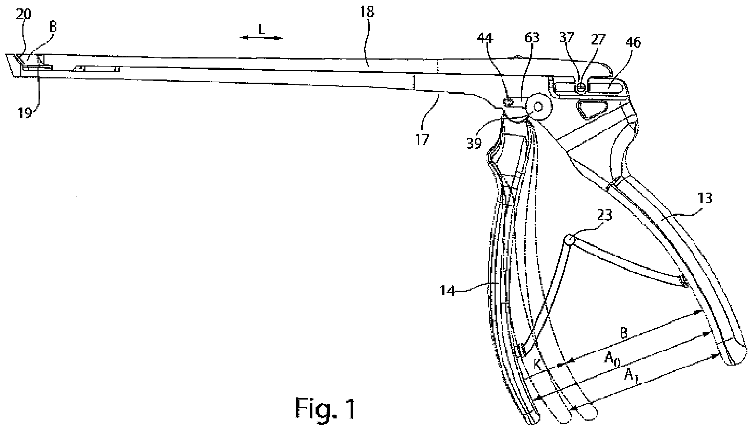

Das chirurgische Instrument gemäß Figur 1 weist einen Schaft 17 auf und einen Schieber 18, der relativ zum Schaft 17 in Längsrichtung L verschiebbar ist. Am distalen Ende des Schafts 17 und des Schiebers 18 können Werkzeugelemente 19, 20 vorgesehen sein. Je nach Anwendungszweck können Art und Konfiguration des Werkzeugs variieren. Beispielsweise können als Werkzeugelemente 19, 20 ein Stanzabschnitt 19 am Schieber 18 und ein Stanzwiderlager 20 am Schaft 17, aber auch scheren-, zangen- oder klemmenartige Schneidwerkzeuge oder Fasswerkzeuge vorgesehen sein.

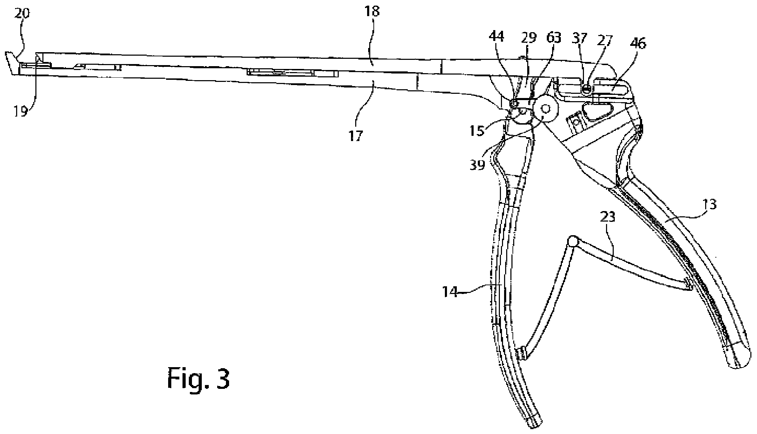

Das proximale Ende des Schafts 17 geht einstückig in einen feststehenden Griff 13 über. Ein weiterer, schwenkbarer Griff 14 ist mittels einer Griffschwenkachse 15 an dem Schaft 17 befestigt (Figur 3), Der schwenkbare Griff 14 und der feststehende Griff 13 sind durch ein Federelement 23 in einer Ausgangsposition zueinander angeordnet, wobei das Federelement 23 die Griffe 13, 14 auseinanderspreizt. In der Ausgangsposition (Abstand A0) sind die Werkzeugelemente 19, 20 zueinander beabstandet, wie dies in Figur 1 dargestellt ist.

Wird nun der schwenkbare Griff 14 betätigt, d.h. in Richtung des Pfeils K in Figur 1 in Richtung des feststehenden Griffs 13 gedrückt, so wird der schwenkbare Handgriff 14 um die Griffschwenkachse 15 geschwenkt und der Abstand A0 (Ausgangsposition) zwischen dem feststehenden Griff 13 und dem schwenkbaren Griff 14 beispielsweise auf den Abstand B (Betätigungsposition) verringert. Da der schwenkbare Griff 14 mit dem Schieber 18 über nachfolgend beschriebene Kopplungsmittel (Schenkel 29, Schieberausnehmung 28) bewegungsgekoppelt ist, wird bei der Betätigung des Griffs 14 in Richtung des Pfeils K der Schieber 18 in Richtung des distalen Endes des Instruments und damit das Werkzeugelement 19 an dem Schieber 18 auf das Werkzeugelement 20 an dem Schaft 17 zu bewegt.

In der Betätigungsposition B, d.h. bei maximal zusammengedrücktem Handgriff (gestrichelt eingezeichnet in Figur 1), liegt das Werkzeugelement 19 (Stanzabschnitt) an dem Werkzeugelement 20 (Stanzwiderlager) an. In dieser Betätigungsposition B wird beispielsweise ein Knochenstück herausgestanzt, vgl. gestrichelt eingezeichnete Position B am distalen Ende des Instruments in Figur 1.

Wird der schwenkbare Handgriff 14 wieder aus der Betätigungsposition (mit Griffabstand B) gelöst, so wird der Schaft 17 aufgrund der die Griffe 13, 14 auseinanderdrückenden Federkraft des Federelements 23 selbständig wieder in die in Figur 1 dargestellte Ausgangsposition mit dem Griffabstand A0 zurückgeführt.

Die Schwenkbewegung des schwenkbaren Griffs 14 um die Griffschwenkachse 15 wird somit in eine Längsbewegung des Schafts 17 in distale Richtung umgewandelt.

Das Instrument umfasst weiterhin eine Einstellvorrichtung 22, die als Anschlag 21 ausgebildet sein kann, wobei der Anschlag 21 gemäß einer ersten Variante derart angeordnet sein kann, dass er die Schwenkbewegung des schwenkbaren Handgriffs 14 begrenzt.

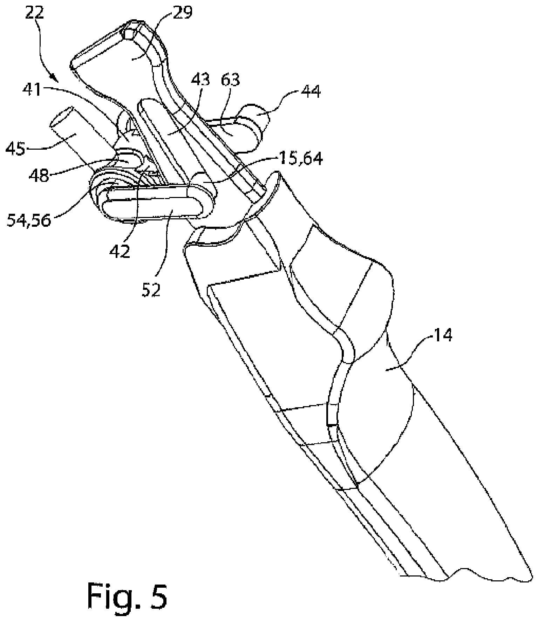

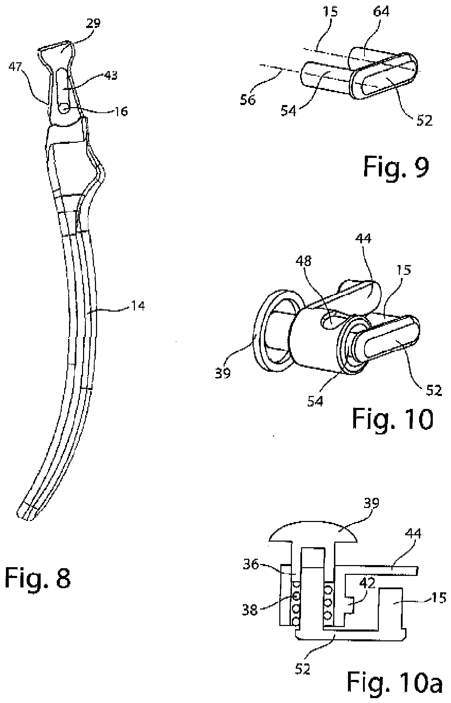

Der Anschlag 21 kann gemäß einer ersten nicht erfindungsgemäßen Ausführungsform als Nocke 42 ausgebildet sein (Figur 5). Vorzugsweise ist die Nocke 42 starr mit einer Hülse 41 verbunden, wobei die Hülse 41 schwenkbar auf einer Hülsenschwenkachse 56 gelagert ist und mit einem von außen bedienbaren Hebel 44 fest verbunden ist. Die Achse des Verbindungsteils 63 zwischen der Hülse 41 und den Hebelgriffs 44 verläuft etwa parallel zu der Seitenwand des Schafts 17 (Figur 1). Zum besseren Verständnis zeigt Figur 5 die Ansicht des schwenkbaren Handgriffs 14 von der rechten Seite, wobei die Schwenkbewegung des schwenkbaren Handgriffs 14 durch Anschlag an die Nocke 42 begrenzt ist. Die Nocke 42 ist starr mit der Hülse 41 verbunden und auf der durch einen nachfolgend näher beschriebenen Entriegelungsbolzen 54 gebildeten Hülsenschwenkachse 56 schwenkbar gelagert ist. Die Hülsenschwenkachse 56 des Entriegelungsbolzens 54 ist in Bezug auf die Griffschwenkachse 15 des schwenkbaren Handgriffs 14 in einem solchen Abstand, dass eine auf der Hülsenschwenkachse 56 des Entriegelungsbolzens 54 oder eines jeden anderen Schwenkbolzens gelagerte Hülse 41 mit Nocke 42 (oder auch andersartig ausgebildete schwenkbare Strukturen mit nicht rotationssymmetrischer Außenfläche wie einem Exzenter) in wenigstens eine Position gebracht werden kann, in der die Schwenkbewegung des schwenkbaren Handgriffs 14 gegenüber einer "neutralen", die Schwenkbewegung des schwenkbaren Handgriffs 14 nicht beschränkenden Bewegung beschränkt werden kann. In der in Figur 5 gezeigten Position liegt die Nocke 42 an der in distale Richtung weisenden Schenkelaußenkante 47 (vgl. Fig. 8) des Schenkels 29 des schwenkbaren Handgriffs 14 an und beschränkt somit den Hub des schwenkbaren Handgriffs 14 und damit auch den Hub des Schiebers 18 und die Öffnung der Werkzeugelemente 19, 20.

Wird der schwenkbare Griff 14 aus der Betätigungsposition B (in Figur 1) nach Drehung des Hebels 44, so dass die Achse des Hebels 44 sich in L-Richtung erstreckt, in die in Figur 1 dargestellte Anschlagposition gebracht und der schwenkbare Griff 14 gelöst, so liegt der schwenkbare Griff 14 an der Nocke 42 an (Figur 5), und es ist eine andere Ausgangsposition A1 mit geänderter Griffweite und Huböffnung eingestellt (Figur 1).

In Abhängigkeit von der Stellung des Hebels 44 sind zwei Positionen der Hülse 41 bzw. der Nocke 42 in Bezug auf die anliegende Schenkelaußenkante 47 des Schenkels 29 des schwenkbaren Griffs 14 möglich. In der "neutralen" Ausgangsposition A0, bei der der Schenkel 29 an der Hülse 41 anliegt, steht das Verbindungsteil 63 des Hebels 44 nach oben, und der Abstand zwischen dem schwenkbaren Handgriff 14 und dem feststehenden Handgriff 13 (und damit der Hub) ist maximal, da die Federkraft des Federelements 23 die Griffe 13, 14 auseinanderspreizt.

Wird nun der schwenkbare Handgriff 14 betätigt, so kann die Hülse 41 mittels des Hebels 44 in eine Position geschwenkt werden, in der die Nocke 42 in Richtung der distalen Schenkelaußenkante 47 des Schenkels 29 gerichtet ist, so dass der Schenkel 29, wenn der schwenkbare Handgriff 14 freigegeben wird, dann vor Erreichen der zuvor beschriebenen neutralen Position A0 an der Nocke 42 anliegt, wobei das Federelement 23 die Griffe 13, 14 auseinanderdrückt (Figur 5). Die durch den Hebel 44 in Anschlagposition gebrachte Nocke 42 verhindert somit, dass der schwenkbare Griff 14 wieder in die Position mit maximaler Griffweite (A0) zurückgelangt. Somit ist durch Betätigung des Hebels 44 die Griffweite in einer - engeren - Ausgangsposition A1 einstellbar, der engere Ausgangsabstand der Griffe 13, 14 entspricht einem geringeren Hub. Die Knochenstanze mit einer auf diese Weise durch Betätigung des Hebels 44 und dadurch Positionierung der entsprechenden Nocke 42 als Anschlag verengten Griffweite und verkürztem Hub kann dann für die Operation bei Knochen geringerer Dicke eingesetzt werden.

Der Hebel 44 ist vorzugsweise mit dem Daumen bedienbar.

An der zylindrischen Außenfläche der Hülse 41 ist eine längliche Hülsenausnehmung 48, in der ein Druckstück 45 in verschiedene Einkerbungen 40 entsprechend bestimmten Anschlagspositionen der Nocke oder Hülse 42 eingerastet werden kann (vgl. Fig. 5, Fig. 10). Durch den Anschlag des Druckstücks 45 an den Kanten der länglichen Hülsenausnehmung 48 ist die Schwenkbewegung des Hebels 44 beschränkt.

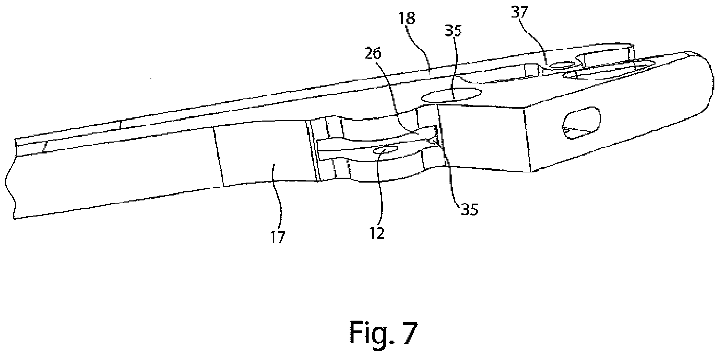

Zur schwenkbaren Lagerung des schwenkbaren Griffs 14 an dem Schaft 17 weist der Schaft 17 einen Durchbruch 26 auf, der in der Schwenkebene liegt (Figur 7). Der schwenkbare Griff 14 greift mit seinem Schenkel 29, der vorzugsweise eine geringere Materialstärke aufweist als der verbleibende Teil des schwenkbaren Griffs 14, durch diesen Durchbruch 26 hindurch, um auf der anderen Seite des Durchbruchs 26 in Eingriff mit dem Schieber 18 stehen zu können. Ein Gelenkzapfen 64, der die Griffschwenkachse 15 bildet, ist im Bereich des Durchbruchs 26 vorgesehen und greift in eine erste Durchgangsöffnung 16 (Figur 7, 8), welche den schwenkbaren Griff 14 durchsetzt, ein, um den schwenkbaren Griff 14 um den die Griffschwenkachse 15 bildenden Gelenkzapfen 64 schwenkbar an dem Schaft 17 zu lagern. Der Gelenkzapfen 64 bildet somit die Griffschwenkachse 15 des schwenkbaren Handgriffs 14.

Der Gelenkzapfen 64 durchquert eine runde Durchgangsbohrung 12 in dem Schaft 17 und greift in ein rundes Sackloch auf der gegenüberliegenden Seite des Schafts ein, so dass der Gelenkzapfen 64 und damit die Griffschwenkachse 15 in Bezug auf eine Bewegung des Schafts in Längsrichtung L ortsfest ist. Die Griffschwenkachse 15 ist somit nicht gegenüber dem Schaft 17 in Längsrichtung verschiebbar.

Der Schenkel 29 des schwenkbaren Handgriffs 14 ist abgerundet ausgebildet und greift in eine Schieberausnehmung 28 des Schiebers 18 ein. Die Schieberausnehmung 28 ist dabei vorzugsweise als Lagerpfanne für den Schenkel 29 geformt, so dass bei Einschieben des Schenkels 29 in die Schieberausnehmung 28 der schwenkbare Griff 14 und der Schieber 18 relativ zueinander positioniert werden. Bei einem Verschwenken des schwenkbaren Griffs 14 nimmt der Schenkel 29 des schwenkbaren Griffs 14 den Schieber 18 über die proximale Schieberausnehmung 28 mit, so dass insgesamt bei einem Verschwenken des schwenkbaren Griffs 14 gegen den feststehenden Griff 13 eine axiale Verschiebung des Schiebers 18 relativ zu dem Schaft 17 erreicht wird.

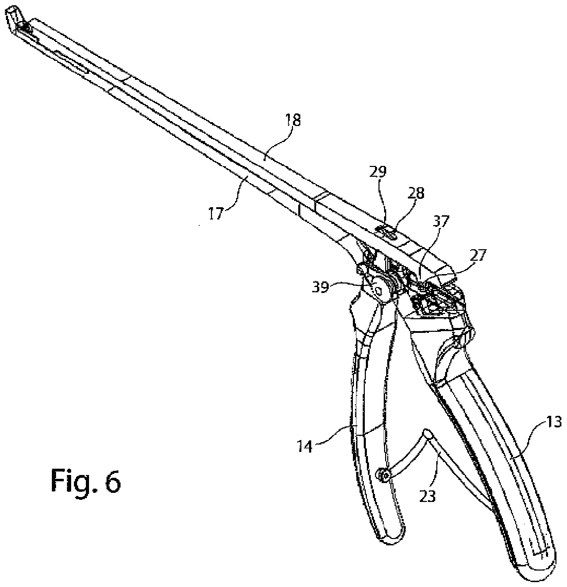

Wie aus Figur 6 ersichtlich, ist bei dem chirurgischen Instrument die Schieberausnehmung 28 als Durchbruch durch den Schieber 18 ausgebildet und somit einfach zu reinigen.

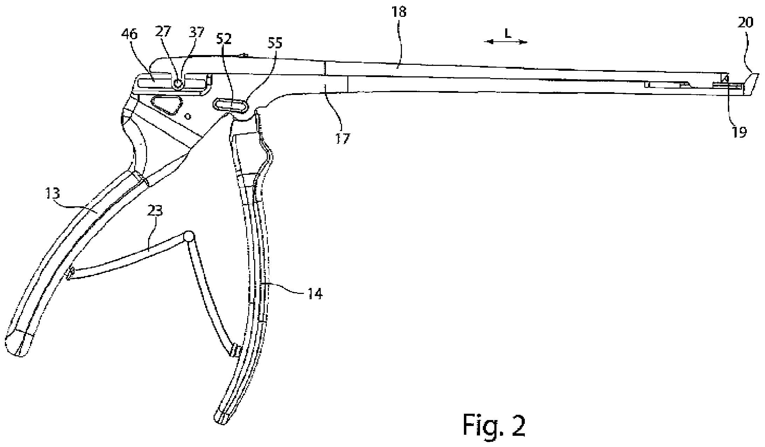

Die axiale Länge des Gelenkzapfens 64, der die Griffschwenkachse 15 bildet, ist so bemessen, dass dieser von einer Seite (rechte Seite) (Figur 2) des Schaftes 17 durch die Durchgangsöffnung 12 des Schafts 17 (rückwärtige Seite in Figur 6) in den Durchbruch 26 eingreifen kann und auch gegen die Gegenseite des Durchbruchs 26 zur Anlage kommt (nicht dargestellt). Hierfür kann die Gegenseite des Durchbruchs 26 eine entsprechend geformte Ausnehmung aufweisen, in welche das freie Ende des Gelenkzapfens 64 eingreifen kann, um den Gelenkzapfen 64 stabil zu lagern.

Der Gelenkzapfen 64, der die Griffschwenkachse 15 des schwenkbaren Handgriffs 14 bildet, ist über ein im Wesentlichen senkrecht zu seiner Achse verlaufendes Joch 52 fest mit einem Entriegelungsbolzen 54 verbunden (Figur 9, 10). Der Entriegelungsbolzen 54 verläuft achsparallel zu dem Gelenkzapfen 64 und ist über das Joch 52 soweit gegen den Gelenkzapfen 64 versetzt und von diesem beabstandet, dass sich der Entriegelungsbolzen 54 außerhalb des Schwenkbereichs des Schenkels 29 des schwenkbaren Griffs 14 in dem Schaft 17 befindet. Die axiale Länge des Entriegelungsbolzens 54 ist so bemessen, dass der Entriegefungsbolzen 54 über den gesamten Schaft 17 hindurchgreift und auf der gegenüberliegenden Fläche aus dem Schaft 17 herausragt (vgl. Figur 1).

Wie bereits zuvor erläutert bildet der Entriegelungsbolzen 54 gleichzeitig die Hülsenschwenkachse 56, auf der die erfindungsgemäße Einstellvorrichtung 22, d.h. die Hülse 41 mit der Nocke 42 und dem Hebel 44, schwenkbar gelagert ist. Generell kann die Hülsenschwenkachse 56 für die Einstellvorrichtung 22 auch ein einfacher Bolzen sein, der ausschließlich als Schwenkachse für die Einstellvorrichtung 22 dient, ohne weitere Funktionen wie die in dieser Ausführungsform beschriebene weitere Funktion der Entriegelung zu erfüllen.

Die Verbindung der Griffschwenkachse 15 des schwenkbaren Handgriffs 14 mit der Hülsenschwenkachse 56 für die Einstellvorrichtung über ein Joch 52 ist für die Verringerung des Hubs des Schiebers 18 bzw. der Handgriffe 13, 14 nicht erforderlich.

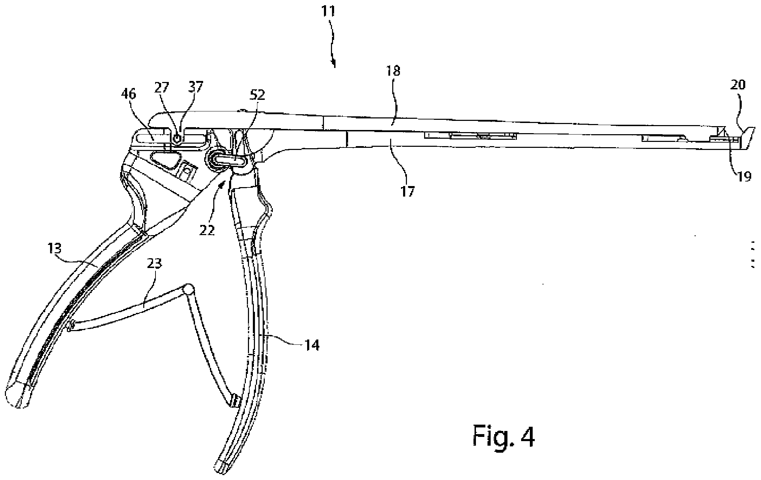

In der rechten Außenfläche des Schaftes 17 gemäß Figur 2 und 4 ist eine Aussparung 55 (nicht dargestellt) vorgesehen, die das Joch 52 oberflächenbündig aufnehmen kann, um bei der Benutzung des chirurgischen Instruments keine unerwünschten Kanten zu bilden. Der Entriegelungsbolzen 54 ist in einer Bohrung 35 des Schafts 17 axial verschiebbar (Figur 7).

Zwischen einem Außenbund 36 des Entriegelungsbolzens 54 und einer Innenschulter der Bohrung 35 ist eine Schraubenfeder 38 eingesetzt, die den Entriegelungsbolzen 54 und mit diesem den Gelenkzapfen 64 in die Verriegelungsstellung vorspannt. Wird der Zerlegeknopf 39 gegen den Druck der Feder 38 gegen den Entriegelungsbolzen 54 in dem Schaft 17 gedrückt, springt das Joch 52 mit dem Entriegelungsbolzen 54 und dem Gelenkzapfen 64 aus dem Durchbruch 26 des Schaftes 17 heraus (vgl. Figur 10a).

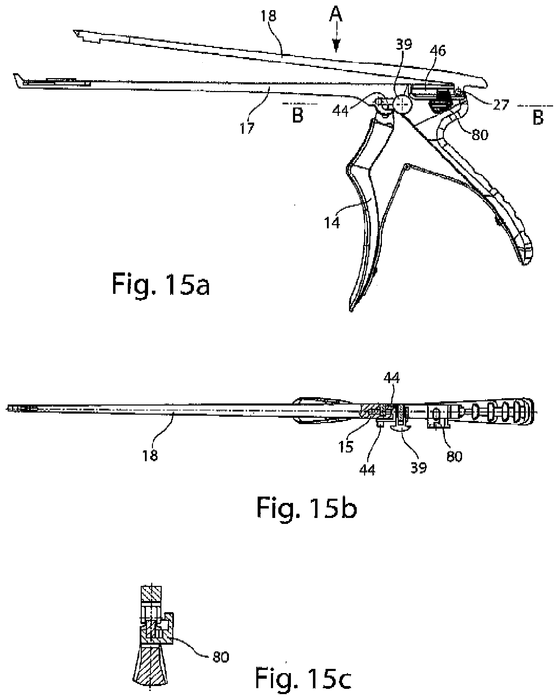

Diese Entriegelungsstellung ist in Figur 15a gezeigt. In dieser Entriegelungsstellung ist es möglich, den schwenkbaren Griff 14 aus dem Durchbruch 26 nach unten herauszuziehen und den Schenkel 29 außer Eingriff mit dem Schieber 18 zu bringen. In dieser Stellung ist es somit möglich, den Schieber 18 von dem Schaft 17 zu Reinigungszwecken zu lösen.

Grundsätzlich ist es möglich, den Schieber 18 vollständig von dem Schaft 17 zu entfernen. Der Schieber 18 bleibt jedoch über eine schwenkbare Verbindung mit dem Schaft 17 verbunden, die durch ein am distalen Ende des Schaftes 18 angeordnetes, in Längsrichtung des Schaftes 17 verlaufendes und quer zur Längsachse des Schaftes 17 den Schaft 17 durchsetzendes Langloch 46 gebildet ist, an dem der Schieber 18 befestigt ist. Die Befestigung erfolgt derart, dass an dem Schieber 18 parallel zueinander und von einander beabstandet zwei Wangen 37 angeordnet sind, die im Wesentlichen an den Seitenflächen des Schaftes 17 anliegen und mittels eines quer zu den Wangen 37 verlaufenden Bolzens 27 verbunden sind. Der Bolzen 27 durchsetzt das Langloch 46, um auf diese Weise die schwenkbare Verbindung zwischen dem Schaft 17 und dem Schieber 18 zu bilden. Der Schaft 17 ist insbesondere im Bereich oberhalb des Langlochs 46 in verringerter Breite ausgebildet, so dass die Wangen 37 die Breite des chirurgischen Instruments nicht vergrößern. Bei einer Relativbewegung des Schiebers 18 gegenüber dem Schaft 17 ist der Schieber 18 über den Bolzen 27 in dem Langloch 46 sicher geführt.

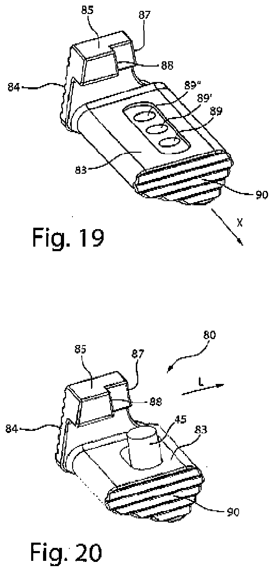

In einer weiteren Variante der Erfindung, die in den Figuren 19 - 23 beschrieben wird, kann in das Langloch 46 eine Einstellvorrichtung, insbesondere ein Riegel in Querrichtung in unterschiedlicher Position eingeschoben werden, um den Schiebeweg des Schiebers 18 zu verkürzen und damit den Hub zu verändern.

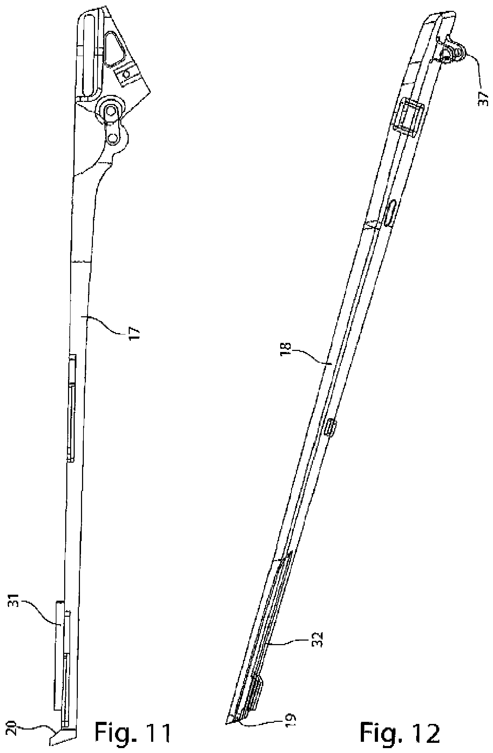

Wird der schwenkbare Griff 14 nach Entriegelung nach unten herausgezogen, greift der Schenkel 29 nicht mehr in die Schieberausnehmung 28 des Schiebers 18, so dass ein Lösen des Schiebers 18 vom Schaft 17 ermöglicht wird. Dieses Lösen erfolgt mittels Verschieben des Schiebers 18 derart, dass im distalen Bereich des Schiebers 18 eine T-förmige Nut 32 außer Eingriff mit einem entsprechenden T-förmigen Element 31 gebracht werden kann (Fig. 11, 12) und anschließend der Schieber 18 um den Bolzen 27 von dem Schaft 17 abgeschwenkt werden kann, so dass eine Reinigung des Bereichs zwischen dem Schieber 18 und dem Schaft 17 auf einfache Weise möglich ist (Figur 15).

Um den schwenkbaren Griff 14 in dieser Reinigungsposition ebenfalls unverlierbar an dem Schaft 17 zu halten, ist in einer der Seitenflächen des bewegbaren Griffs 14 ausgehend von der ersten Durchgangsöffnung 16 eine Nut 43 angeordnet (Figur 5, 8), in welche das freie Ende des Gelenkzapfens 64 nach Überführung in die Entriegelungsstellung und geringfügigem Herausziehen des schwenkbaren Griffs 14 aus dem Schaft 17 eingreift.

Die Nut 43 bildet eine Führung für den Gelenkzapfen 64 während der Bewegung des schwenkbaren Griffs 14 beim Herausziehen des schwenkbaren Griffs 14 aus dem Schaft 17. Weiterhin bildet das der ersten Durchgangsöffnung 16 abgewandte Ende der Nut 43 einen Anschlag für den Gelenkzapfen 64 bei dieser Bewegung, so dass der bewegbare Griff 14 nicht weiter aus dem Schaft 17 herausgezogen werden kann und in dieser Position unverlierbar an dem Schaft 17 gehalten ist. Gleichzeitig wird jedoch der schwenkbare Griff 14 derart weit aus dem Schaft 17 herausgezogen, dass der Schenkel 29 des schwenkbaren Griffs 14 von dem Schaft 17 getrennt ist und somit außer Eingriff mit dem Schaft 17 ist, so dass der Schieber 18 ungehindert wie zuvor beschreiben von dem Schaft 17 gelöst werden kann. Auf diese Weise wird eine besonders einfache Verliersicherung des schwenkbaren Griffs 14 an dem Schaft 17 ermöglicht und der Schieber 18 unabhängig von dem schwenkbaren Griff 14 an dem Schaft 17 gehalten.

In dieser Ausführungsform kann die Zerlegung des Instruments in jeder Position des Hebels 44 erfolgen.

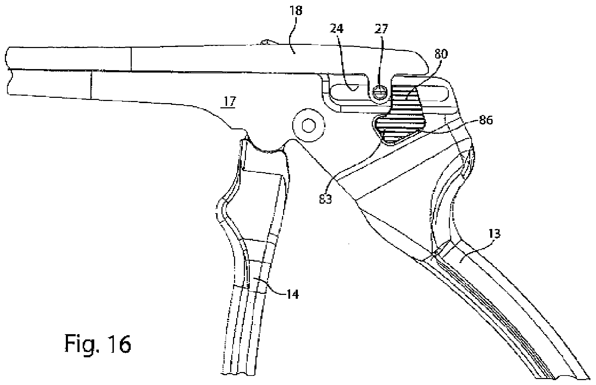

In einer weiteren Ausführungsform, die in Figur 16 dargestellt ist, ist die Einstellvorrichtung 22 eine Scheibe 50, die außerhalb ihres Mittelpunktes M und somit exzentrisch in dem Durchbruch 26 innerhalb des Schaftes 17 auf einer die Wand 49 des Schaftes 17 durchquerende Drehachse 51 befestigt ist. Die Scheibe 50 ist rechteckig. Je nach Drehposition der exzentrischen Scheibe 50 lässt sich ein anderer Abstand zwischen der distalen Schenkelaußenkante 47 des Schenkels 29 des schwenkbaren Handgriffs 14 und den Exzenterscheibenaußenkanten 57, 58, 59, 60 der Scheibe 50 einstellen.

Die Drehachse 51 der Scheibe 50 ist achsparallel der Griffschwenkachse 15 des schwenkbaren Handgriffs 14 und in einem solchen Abstand angeordnet, dass die in verschiedene Positionen drehbare Scheibe 50 mit ihren Exzenterscheibenaußenkanten 57, 58, 59, 60 in unterschiedlichen Abständen zu der Griffschwenkachse 15 des schwenkbaren Handgriffs 14 und damit in unterschiedliche Anschlagpositionen positioniert werden kann.

Figur 16 zeigt die erfindungsgemäße Ausführungsform, bei der die Einstellvorrichtung 22 in Form eines Verschieberiegels 80 ausgebildet ist, der in Querrichtung in die proximale Schaftausnehmung (Langloch) 24 in den Schaft 17 hineingeschoben werden kann.

Der Riegel 80 ist dabei so ausgebildet, dass in Abhängigkeit davon, wie weit er in Querrichtung in das Langloch 24 hinreicht, unterschiedlich weit vom proximalen Ende des Langlochs 24 beabstandete Flächen 87, 88 einen Anschlag für den Bolzen 27 des Schiebers 18 bilden. Je nachdem, wie weit der Riegel 80 in den Verschiebeweg des Bolzens 27 in dem Langloch 24 hineingeschoben ist, ist der Hub verkürzt, stärker verkürzt oder nicht verkürzt.

In der einfachsten Variante weist der Verschieberiegel 80 nur eine einzige Anschlagfläche auf, die in Abhängigkeit von der Verschiebeposition des Riegels 80 entweder in Anschlagposition gebracht werden kann und somit den Hub verkürzt oder aber außer Anschlag gebracht werden kann entsprechend dem maximalen Hub.

Der Verschieberiegel 80 umfasst ein oberes Teilstück 85, das die Anschlagsfläche 87, 88 aufweist und das letztendlich die Verriegelung bewirkt, ein Mittelstück 84 und ein unteres Teilstück 83. Das untere Teilstück 83 des Verschieberiegels 80 ist in die Öffnung 86 unterhalb des Langlochs 24 in dem Schaft 17 verschiebbar hineingesteckt. Das untere Teilstück 83 ist in der Öffnung 86 geführt. Unteres Teilstück 83 und Öffnung 86 sind dreieckig zylindrisch ausgebildet, es sind jedoch auch andere Formen möglich. Die Anschlagsflächen 87, 88 sind stufenförmig angeordnet. Auf der nach oben weisenden Seite des unteren Teils 83 sind in Querrichtung verlaufend drei Näpfe 89, 89', 89", in die ein federgelagertes Rastelement 45 eingreifen kann.

Befindet sich das Rastelement 45 in dem am weitesten von dem Mittelstück 84 entfernte Napf 89, entspricht dies einer ersten Position, nämlich der Position, in der der Verschieberiegel 80 zwar in die Öffnung hineingesteckt ist, die Längsbewegung des Bolzens 27 jedoch noch nicht beeinträchtigt wird, d.h. in dieser ersten Position entsprechend dem Druckelement 45 in dem Napf 89 schlägt der Bolzen 27 bei der Längsbewegung in L-Richtung noch nicht an einer Anschlagfläche an.

Wird der Verschieberiegel 80 nun weiter in die Öffnung 86 hineingeschoben, so rastet das Druckelement 45 in dem zweiten Napf 89' ein. Diese Position entspricht einer Position, in der die Stufe 88 mit ihrer in distale Richtung des Instruments weisenden Kante in den Bereich des Langlochs 24, in dem die Bewegung des Bolzens 27 erfolgt, hineinreicht, so dass die Stufe 88 den Hub des Bolzens 27 bzw. des Schiebers 18 verkürzt.

In dieser Position steht bereits das Endstück 90 des unteren Teilstücks 83 auf der rechten Seite des Schaftes 17 etwas von der Außenseite des Schaftes 17 hervor. Durch Drücken gegen dieses Endstück 90 des unteren Teilstücks 83 kann der Riegel 80 wieder aus der ersten Anschlagposition "entriegelt" werden.

Wird der Verschieberiegel 80 weiter in die Öffnung 86 hineingeschoben, so rastet das Druckstück 45 in dem dritten Napf 89" ein. Bei einer Verschiebebewegung des Bolzens 27 schlägt dieser nun bereits an der in den Bewegungsraum hineinreichenden Stufe 87 an, die den Hub gegenüber der Stufe 88 weiter verkürzt. Selbstverständlich können auch mehr Näpfe 89 und mehr Stufen 87 vorgesehen sein, aber auch weniger.

Auch hier erfolgt die Entriegelung durch Druck auf das aus der Öffnung 86 in dem Schaft 17 hervorstehende Endstück 90.

Um das Instrument dieser Ausführungsform zum Reinigen zu zerlegen, muss der Riegel 80 vollständig aus dem Verschiebeweg des Bolzens 27 entfernt werden, da der Bolzen 27 ansonsten nicht in die zum Zerlegen erforderliche Position am distalen Ende des Langlochs 24 geschoben werden kann (vgl. Figur 15c).

## Patentansprüche

1. Chirurgisches Instrument mit einem Schaft (17), der proximal in einen feststehenden Handgriff (13) mündet und einem Schieber (18), der auf dem Schaft (17) in Längsrichtung verschiebbar ist, wobei der Schieber (18) mit einem schwenkbaren Handgriff (14) bewegungsgekoppelt ist, wobei der schwenkbare Handgriff (14) mit dem feststehenden Handgriff (13) gelenkig verbunden ist, wobei an dem Schieber (18) und an dem Schaft (17) Werkzeugelemente (19, 20) vorgesehen sind, die zum Schneiden, Stanzen, Durchtrennen oder Fassen dienen, wobei eine Einstellvorrichtung (22) vorgesehen ist, die zwischen dem feststehenden Handgriff (13) und dem schwenkbaren Handgriff (14) wirkt und die Hubbewegung des schwenkbaren Handgriffs (14) zu dem feststehenden Handgriff (13) aus einer Betätigungsposition in eine Ausgangsposition mit verschiedenen Griffweiten einstellt, und wobei am proximalen Ende des Schafts (17) eine proximale Schaftausnehmung (24), insbesondere ein Langloch, vorgesehen ist, in der ein mit dem Schieber (18) verbundener Bolzen (27) verschiebbar geführt ist, **dadurch gekennzeichnet, dass** der Bolzen (27) starr mit dem Schieber (18) verbunden ist.

2. Chirurgisches Instrument nach Anspruch 1, **dadurch gekennzeichnet, dass** die Einstellvorrichtung (22) ein in der proximalen Schaftausnehmung (24) in den Schaft (17) in Querrichtung verschiebbarer Riegel (80) ist, der eine Anschlagfläche oder mehrere stufenförmige Anschlagflächen (87, 88) aufweist, wobei im Falle von mehreren stufenförmigen Anschlagflächen (87, 88) die Anschlagflächen (87, 88) unterschiedlich weit in Querrichtung in die proximale Schaftausnehmung (24) hineinreichen, und die eine oder mehrere Anschlagflächen (87, 88) in Abhängigkeit von der Verschiebeposition des Riegels (80) als Anschlag für den Bolzen (27) dienen.

3. Chirurgisches Instrument nach Anspruch 2, **dadurch gekennzeichnet, dass** in dem Schaft (17) unterhalb der proximalen Schaftausnehmung (24) eine Öffnung (86) vorgesehen ist, in der der Riegel (80) in Querrichtung verschiebbar ist.

4. Chirurgisches Instrument nach einem der Ansprüche 1 bis 3, **dadurch gekennzeichnet, dass** der feststehende Handgriff (13) mit dem schwenkbaren Handgriff (14) über einen Gelenkzapfen (64) verbunden ist, der in Bezug auf eine Bewegung entlang der Längsachse L des Schafts (17) in einer ortsfesten Position ist.

5. Chirurgisches Instrument nach einem der Ansprüche 1 bis 4, **dadurch gekennzeichnet, dass** der Schenkel (29) des schwenkbaren Handgriffs (14) in eine Schieberausnehmung (28) in dem Schieber (18) eingreift, wobei die Schieberausnehmung (28) vorzugsweise als Lagerpfanne für den Schenkel (29) geformt ist.

6. Chirurgisches Instrument nach Anspruch 5, **dadurch gekennzeichnet, dass** die Schieberausnehmung (28) als Durchbruch durch den Schieber (18) ausgebildet ist.

7. Chirurgisches Instrument nach einem der Ansprüche 1 bis 6, **dadurch gekennzeichnet, dass** der schwenkbare Griff (14) durch einen Durchbruch (26) im Schaft (17) hindurch in Eingriff mit dem Schieber (18) ist und der Schieber (18) über einen Bolzen (27) in einer proximalen Schaftausnehmung (24) in den Schaft (17) geführt ist.

8. Chirurgisches Instrument nach einem der Ansprüche 1 bis 7, **dadurch gekennzeichnet, dass** im distalen Bereich des Schiebers (18) eine T-förmige Nut (32) vorgesehen ist, die in Eingriff mit einem korrespondierenden T-förmigen Element (31) im distalen Bereich des Schafts (17) gebracht werden kann.

9. Chirurgisches Instrument nach einem der Ansprüche 1 bis 8, **dadurch gekennzeichnet, dass** ein achsparallel zu dem Gelenkzapfen (64) verlaufender Entriegelungsbolzen (54) vorgesehen ist.

10. Chirurgisches Instrument nach Anspruch 9 und einem der Ansprüche 1 und 4 bis 8, **dadurch gekennzeichnet, dass** der Entriegelungsbolzen (56) die Drehachse (56) der Hülse (41) ist.

## Claims

1. A surgical instrument having a shaft (17) which on a proximal side joins a fixed handle (13) and a slide (18) which is displaceable on the shaft (17) in a longitudinal direction, said slide (18) being coupled for movement with a pivoting handle (14),
wherein
the pivoting handle (14) is hingedly connected with the fixed handle (13), wherein the slide (18) and the shaft (17) have end tool elements (19, 20) provided thereon that serve for cutting, punching, severing, or grasping, wherein a setting device (22) is provided which acts between the fixed handle (13) and the pivoting handle (14) in order to set the travel of the pivoting handle (14) with respect to the fixed handle (13) at different grip widths when moving from an actuating position to an initial position, and
wherein
on the proximal end of the shaft (17), a proximal shaft recess (24), in particular an elongated hole, is provided in which a pin (27) connected with the slide (18) is guided for displacement, **characterised in that** said pin (27) is rigidly connected to said slide (18).

2. The surgical instrument as claimed in claim 1, **characterised in that** the setting device (22) is a bolt (80) displaceable within the proximal shaft recess (24) in a transverse direction into the shaft (17) and having a stop surface or several stepped stop surfaces (87, 88), said stop surfaces (87, 88), if several stepped stop surfaces (87, 88) are provided, extending different distances into the proximal shaft recess (24) in a transverse direction and said stop surface or plurality of stop surfaces (87, 88) serving as a stop for the pin (27), depending on the position of displacement of the bolt (80).

3. The surgical instrument as claimed in claim 2, **characterised in that** the shaft (17) has an opening (86) provided beneath the proximal shaft recess (24) wherein the bolt (80) is displaceable in a transverse direction.

4. The surgical instrument as claimed in any one of claims 1 to 3, **characterised in that** the fixed handle (13) and the pivoting handle (14) are interconnected via a pivot pin (64) which is in a stationary position relative to a movement along the longitudinal axis L of the shaft (17).

5. The surgical instrument as claimed in any one of claims 1 to 4, **characterised in that** the branch (29) of the pivoting handle (14) engages with a slide recess (28) provided in the slide (18), said slide recess (28) being preferably shaped in the form of a bearing cup for the branch (29).

6. The surgical instrument as claimed in claim 5, **characterised in that** the slide recess (28) is realised as an aperture traversing the slide (18).

7. The surgical instrument as claimed in any one of claims 1 to 6, **characterised in that** the pivoting handle (14) engages with the slide (18) through an aperture (26) provided in the shaft (17) and **in that** within a proximal shaft recess (24) the slide (18) is guided into the shaft (17) via a pin (27).

8. The surgical instrument as claimed in any one of claims 1 to 7, **characterised in that** in the distal region of the slide (18) a T-shaped groove (32) is provided which may be engaged with a corresponding T-shaped element (31) formed in the distal region of the shaft (17).

9. The surgical instrument as claimed in any one of claims 1 to 8, **characterised in that** a release pin (54) is provided the axis of which extends parallel with that of the pivot pin (64).

10. The surgical instrument as claimed in claim 9 and in any one of claims 1 and 4 to 8, **characterised in that** the release pin (56) constitutes the axis of rotation (56) of the sleeve (41).

## Revendications

1. Instrument chirurgical pourvu d'une tige (17) qui débouche, coté proximal, dans une poignée fixe (13) et d'un coulisseau (18) qui peut coulisser en sens longitudinal sur ladite tige (17), le coulisseau (18) étant couplé à une poignée pivotante (14) de manière à se déplacer avec celle-ci,
dans lequel
la poignée pivotante (14) est reliée de manière articulée à la poignée fixe (13), dans lequel des éléments-outils (19, 20) qui servent à couper, poinçonner, séparer ou saisir sont prévus sur le coulisseau (18) et sur la tige (17), dans lequel il est prévu un dispositif de réglage (22) qui agit entre la poignée fixe (13) et la poignée pivotante (14) et règle la course qu'effectue la poignée pivotante (14) par rapport à la poignée fixe (13) sur différentes ouvertures de poignées, lorsqu'elle passe d'une position d'actionnement à une position initiale, et
dans lequel
à l'extrémité proximale de la tige (17) est prévu un évidement proximal de tige (24), en particulier un trou oblong, dans lequel un boulon (27) relié au coulisseau (18) est déplacé de manière guidée, **caractérisé en ce que** le boulon (27) est relié de manière rigide au coulisseau (18).

2. Instrument chirurgical selon la revendication 1, **caractérisé en ce que** le dispositif de réglage (22) est un verrou (80) pouvant être déplacé dans la tige (17), en sens transversal dans l'évidement proximal de tige (24) et présentant une surface d'arrêt ou plusieurs surfaces d'arrêt (87, 88) disposées en gradins, lesdites surfaces d'arrêt (87, 88), s'il y a plusieurs surfaces d'arrêt (87, 88) disposées en gradins, s'étendant en sens transversal jusqu'à différentes profondeurs dans l'évidement proximal de tige (24) et ladite surface d'arrêt ou pluralité de surfaces d'arrêt (87, 88) servant de butée au boulon (27) en fonction de la position de coulissement du verrou (80).

3. Instrument chirurgical selon la revendication 2, **caractérisé en ce que** dans la tige (17), au-dessous de l'évidement proximal de tige (24), est prévue une ouverture (86) dans laquelle le verrou (80) peut coulisser en sens transversal.

4. Instrument chirurgical selon l'une quelconque des revendications 1 à 3, **caractérisé en ce que** la poignée fixe (13) est reliée à la poignée pivotante (14) par un tourillon d'articulation (64) qui se trouve dans une position fixe par rapport à un déplacement le long de l'axe longitudinal L de la tige (17).

5. Instrument chirurgical selon l'une quelconque des revendications 1 à 4, **caractérisé en ce que** la branche (29) de la poignée pivotante (14) entre en prise avec un évidement de coulisseau (28) ménagé dans le coulisseau (18), l'évidement de coulisseau (28) étant réalisé de préférence en tant que coussinet pour ladite branche (29).

6. Instrument chirurgical selon la revendication 5, **caractérisé en ce que** l'évidement de coulisseau (28) est réalisé en tant qu'ouverture traversant le coulisseau (18).

7. Instrument chirurgical selon l'une quelconque des revendications 1 à 6, **caractérisé en ce que** la poignée pivotante (14) se trouve en prise avec le coulisseau (18) grâce à une ouverture (26) traversant de part en part la tige (17) et que le coulisseau (18) est guidé par un boulon (27) dans la tige (17), dans un évidement proximal de tige (24).

8. Instrument chirurgical selon l'une quelconque des revendications 1 à 7, **caractérisé en ce que** dans la zone distale du coulisseau (18) est prévue une rainure (32) en forme de T qui peut être mise en prise avec un élément (31) en forme de T correspondant prévu dans la zone distale de la tige (17).

9. Instrument chirurgical selon l'une quelconque des revendications 1 à 8, **caractérisé en ce qu'**il est prévu un boulon de déverrouillage (54) dont l'axe s'étend parallèlement à celui du tourillon d'articulation (64).

10. Instrument chirurgical selon la revendication 9 et selon l'une quelconque des revendications 1 et 4 à 8, **caractérisé en ce que** le boulon de déverrouillage (56) constitue l'axe de rotation (56) du manchon (41).
